Europäisches Patentamt

European Patent Office

Office européen des brevets

(19)

(11) Publication number: **0 367 447**
**A1**

## EUROPEAN PATENT APPLICATION

(21) Application number: 89310740.9

(22) Date of filing: 19.10.89

(51) Int. Cl.5: **C07K 15/00 , C07K 3/20 , A61K 37/02 , C12P 21/00 , C12P 21/08 , C12N 15/16 , C12N 1/00**

Claims for the following Contracting States: ES + GR.

(30) Priority: **20.10.88 US 260148**

(43) Date of publication of application:
**09.05.90 Bulletin 90/19**

(84) Designated Contracting States:
**AT BE CH DE ES FR GB GR IT LI LU NL SE**

(71) Applicant: **SMITHKLINE BEECHAM CORPORATION**
**One Franklin Plaza**
**Philadelphia Pennsylvania 19103(US)**

(72) Inventor: **Dunnington, Damien John**
**500 E. Lancaster Avenue**
**St. Davids Pennsylvania 19087(US)**
Inventor: **Anzano, Mario Arquillano**
**1709 Mountainview Drive**
**Wayne Pennsylvania 19087(US)**

(74) Representative: **Giddings, Peter John, Dr. et al**
**Smith Kline & French Laboratories Ltd.**
**Corporate Patents Mundells**
**Welwyn Garden City Hertfordshire AL7 1EY(GB)**

(54) Human epithelial transforming growth factor.

(57) This invention relates to a human epithelial transforming growth factor (h-TGFe), a pharmaceutical composition comprising an effective amount of h-TGFe and a pharmaceutically acceptable carrier or diluent, and its use in therapy in stimulating the growth of epithelial cells.

EP 0 367 447 A1

## HUMAN EPITHELIAL TRANSFORMING GROWTH FACTOR

### BACKGROUND OF THE INVENTION

This invention relates to a human epithelial cell transforming growth factor (hereinafter referred to as "h-TGFe"), a pharmaceutical composition comprising an effective amount of h-TGFe and a pharmaceutically acceptable carrier or diluent, a method of stimulating the growth of epithelial cells in a human in need thereof by administering an effective amount of h-TGFe to such human, a method of producing h-TGF-e and a process of purifying h-TGFe.

A number of biologically active polypeptides designated "transforming growth factors" (TGF's) have been identified and isolated by their ability to promote anchorage independent growth of an appropriate indicator cell. Examples include TGF-$\alpha$ - [Marquardt et al., Science, 223, 1079-1083 (1976)], a molecule closely related to epidermal growth factor, and TGF-$\beta$ [Frolick et al., Proc. Natl. Acad. Sci., U.S.A., 80, 3676-3680 (1983)], a substance distinct from TGF-$\alpha$ and other known growth factors. The indicator cells used to measure the activity of these TGF's are of fibroblast origin (NRK-49F rat kidney fibroblasts for TGF-$\alpha$ and $\beta$, and AKR-2B mouse embryo fibroblasts for TGF-$\beta$ only). The use of fibroblast cells in bioassays to detect TGF activity tends to result in the discovery of molecules that are primarily active on mesenchymal cells, although TGF-$\alpha$ has been shown to stimulate the growth of mammary epithelial cells [Perroteau et al., Anticancer Research, 6, 339 (1986)] and TGF-$\beta$ inhibits proliferation of a number of epithelial cell lines [Roberts et al., Proc. Natl. Acad. Sci., U.S.A., 82, 119-123 (1985)].

Halper et al., Cancer Research, 43, 1972-1979 (1983), report that SW13 cells, which were derived from a human adenocarcinoma of the adrenal cortex, formed only a few small colonies when suspended in soft agar at low cell densities, but that the number and size of the colonies increased dramatically following stimulation with serum-free medium conditioned by the SW13 cells, and Halper et al. postulate that such increase indicates the possibility of autostimulation in these malignant cells. Halper et al. also report that acid ethanol extracts and conditioned media from three human carcinoma cell lines (A431, D562 and A549) stimulated SW13 cells to form into progressively growing colonies in soft agar. Preliminary characterization of the active component of acid ethanol extracts of human carcinomas indicated that the active component was acid and heat stable, sensitive to trypsin or dithiothreitol, had an apparent molecular mass of 20-22 kilodaltons and could be separated from TGF-$\alpha$ and $\beta$-like activities by high pressure liquid chromatography (HPLC). Activity mimicking the effects of the acid ethanol extract derived substance was detected in acid ethanol extracts of 26 out of 32 (80%) freshly excised human carcinomas but in only one of 9 non-carcinomatous neoplasms. In addition, activity mimicking the effects of the acid ethanol extract derived substance was detected in extracts of non-neoplastic human and bovine kidney, and to a lesser extent, in human lung and mouse embyros. Halper et al., postulate that the acid ethanol extract derived substance is of epithelial origin.

Halper et al., Cancer Research, 47, 4552-4559 (1987), disclose the purification and characterization of a transforming growth factor isolated from acid ethanol extracts of bovine kidney and state that such bovine derived material (a) stimulated soft agar growth of certain epithelial cell lines such as SW13 cells; two human squamous cell carcinoma cells lines, A431 and D562; mouse embryo AKR-2B cells; (b) had a molecular weight of 23,000 to 25,000 as determined by sodium dodecyl sulfate-polyacrylamide gel electrophoresis (SDS-PAGE); (c) did not bind to the FGF receptor as determined in the FGF radioreceptor binding assay using BHK-21 cells; (d) was heat stable; (e) was labile to trypsin; (f) was probably a single chain polypeptide; (g) was only partially sensitive to reducing agents; and (h) did not bind significantly to either TGF$\beta$ or EGF receptors.

### SUMMARY OF THE INVENTION

This invention relates to a human epithelial cell transforming growth factor (h-TGFe) which has the following characteristics:

a) stimulates anchorage independent growth of SW13 cells in soft agar in an autocrine fashion;

b) is stable to each of pH 2-4, 8M urea and 0.01% Tween 80, but labile to each of 50 $\mu$g/mL trypsin, 0.065M dithiothreitol 0.1 g/100 mL tetrabutylammonium phosphate and 2% sodium dodecyl sulfate;

c) does not bind to heparin-sepharose conjugate but does bind to red sepharose;

d) fails to stimulate the growth of fetal bovine heart endothelial cells in monolayer culture;

e) has a molecular mass of approximately 59 kilodaltons (kD) as measured by size exclusion gel permeation chromatography in the presence of 8M urea;

f) has an isoelectric point of approximately 8.5-10 as measured by cation exchange chromatography in the presence of 8M urea:

g) elutes at 20-30% acetonitrile from a C18 reverse phase column; and

h) is produced by SW13, SW403, LOVO, NRK-52E, WIDR, HCT-15, SKC01 and SW48 cell lines and is detectable in human milk.

By the term 'anchorage independent growth of SW13 cells in soft agar in an autocrine fashion' is meant that the anchorage independent growth of the SW13 cells in soft agar is stimulated by a growth factor produced by the SW13 cells. Such 'anchorage independent growth of SW13 cells in soft agar is measured by the method described by Halper et al., Cancer Research, 43, 1972-1979 (1983). Preferably such method is modified so that the base and upper layers contain 0.5% and 0.3% agarose, respectively in DMEM, 10% fetal bovine serum, and 5000 cells are plated per 3 cm dish.

By the term 'red sepharose' is meant red dye (Procion red HE-3B) attached to agarose beads for support. Red sepharose is commercially available from Pharmacia, Piscataway, New Jersey. Sepharose® is the tradename for the brand of agarose beads sold by Pharmacia.

By the term 'growth of fetal bovine heart endothelial cells in monolayer culture' is meant the growth of such cells as measured by the method of Gospodarowicz et al., J. Biol. Chem., 250, 2515 (1975).

This invention also relates to a pharmaceutical composition comprising an effective amount of h-TGFe and a pharmaceutically acceptable carrier or diluent.

This invention also relates to a method of stimulating the growth of epithelial cells in a human in need thereof which comprises administering an epithelial cell stimulating amount of h-TGFe to such human.

This invention also relates to a method of producing h-TGFe which comprises:

a) culturing, in the absence of serum, cells or a cell line which is capable of producing h-TGFe in conditioned medium in the absence of serum; and

b) isolating the h-TGFe produced from such culturing. Preferably, the method of producing h-TGFe includes the following steps:

a) culturing, in the absence of serum, cells or a cell line which is capable of producing h-TGFe in conditioned medium in the absence of serum;

b) collecting the conditioned medium produced in step a after the cells have grown to confluence;

c) sequentially subjecting the conditioned medium to reverse phase chromatography, size exclusion chromatography and cation exchange high pressure liquid chromatography; and

d) collecting the fractions containing h-TGFe. Moreover, this invention relates to the h-TGFe purified according to the method of this invention.

This invention also relates to a method of purifying h-TGFe which comprises:

a) collecting conditioned medium of cells capable of producing h-TGFe after the cells have grown to confluence;

b) sequentially subjecting the conditioned medium to reverse phase chromatography, size exclusion chromatography and cation exchange high pressure liquid chromatography; and

c) collecting the fractions containing h-TGFe. Moreover, this invention relates to the h-TGFe purified according to the method of this invention.

This invention also relates to a recombinant DNA molecule, such as a cDNA, containing the h-TGFe coding sequence. The term 'h-TGFe coding sequence' encompasses the wild-type h-TGFe coding sequence as well as all functional mutants and derivatives of the native h-TGFe coding sequence. By the term 'functional mutants and derivatives of the native h-TGFe coding sequence' is meant any coding sequence which encodes a protein which has substantially the same biological activity as native h-TGFe, i.e., a protein which has the ability to induce the growth of epithelial cells as described above. Such functional mutants and derivatives of the native h-TGFe coding sequence include chemically modified forms thereof, e.g., glycosylated and alkylated forms which have substantially the same biological activity as native h-TGFe. They also include mutants and derivatives in which one or more amino acids have been rearranged, added, deleted or substituted or in which a heterologous polypeptide has been fused or conjugated thereto.

This invention also relates to a synthetic DNA molecule containing the h-TGFe coding sequence. As stated above, the term 'h-TGFe coding sequence' encompasses the wild-type h-TGFe coding sequence as well as all functional mutants and derivatives of the native h-TGFe coding sequence. By the term 'functional mutants and derivatives of the native h-TGFe coding sequence' is meant any coding sequence which encodes a protein which has substantially the same biological activity as native h-TGFe, i.e., a protein which has the ability to induce the growth of epithelial cells. Such functional mutants and derivatives of the h-TGFe coding sequence include chemically modified forms thereof, e.g., glycosylated and alkylated forms which have substantially the same biological activity as native h-TGFe.

This invention also relates to a recombinant DNA vector containing the h-TGFe coding sequence operatively associated with an expression control sequence.

This invention also relates to a host cell transformed with the vector of this invention, and to a method of producing such a transformed host which comprises transforming a desired host cell with the vector of the invention.

This invention also relates to yet another method of producing h-TGFe which comprises culturing the transformed host of the invention in appropriate culture medium. This invention also relates to the h-TGFe produced according to such method.

The present invention also relates to a method of producing the protein encoded by the coding sequence of this invention which comprises introducing the coding sequence of this invention into mammalian embryos, preferably in association with signal sequences that direct the secretion of h-TGFe into milk, growing the embryos to maturity and inducing them to produce milk, and isolating the protein so produced.

This invention also relates to a polyclonal or monoclonal antibody which is reactive against h-TGFe.

This invention also relates to a polyclonal or monoclonal antibody which is reactive against an h-TGFe antibody. Such a polyclonal or monoclonal antibody is commonly referred to as an anti-idiotype antibody.

## DETAILED DESCRIPTION OF THE INVENTION

This invention relates to a human epithelial transforming growth factor ("h-TGFe") which is distinct from other known growth factors. It has been discovered that h-TGFe stimulates the growth of SW13 cells in soft agar in an autocrine fashion. The SW13 cell line is publicly available from various sources, e.g., from the American Type Culture Collection, Rockville, Maryland, U.S.A., under accession number CCL105.

h-TGFe activity is also found in extracts of human colon carcinomas and is also detectable in acidified extracts of human milk and in serum-free media conditioned by the following cell lines, most of which were derived from colon carcinomas, i.e., SW403, LOVO, NRK-52E, WIDR, HCT-15, SKCO1 and SW48. All of the above cell lines are publicly available. e.g., all of such cell lines are available from the American Type Collection, Rockville, Maryland, U.S.A. These findings suggest an association of h-TGFe activity with epithelial cells and tissues.

The growth stimulation response of SW13 cells to h-TGFe is not mimicked by any of the known growth factors with the exception of fibroblast growth factor (FGF). A commercially available preparation of FGF was used to evaluate the differences between FGF and h-TGFe. Since the commercial preparation of FGF used to test the response of SW13 cells thereto was impure, the FGF material was fractionated by reverse phase HPLC and co-purification of SW13 and fetal bovine heart endothelial cell (FBHE)-stimulatory activity was observed, suggesting that the SW13 cells were indeed responding to FGF and not to an impurity in the sample. Growth of FBHE cells in monolayer culture was performed according to the method of Gospodarowicz et al., J. Biol. Chem., 250, 2515 (1975). However, the factor produced by SW13 cells (h-TGFe) is not identical to FGF since it does not bind to heparin as FGF does, it is acid-stable while FGF is not, and it does not stimulate proliferation of FBHE or 3T3 cells, both of which respond to FGF.

Biochemical characterization of h-TGFe indicates that it is stable to each of the following conditions: pH 2-4, 8M urea and 0.01% Tween 80, but that h-TGFe is labile to each of the following conditions: 2% sodium dodecyl sulfate, 50 µg/mL trypsin, 0.1 g/100 mL tetrabutylammonium phosphate and 0.065M dithiothreitol. The molecular mass estimated by size exclusion gel permeation chromatography in the presence of 8M urea is approximately 59 kilodaltons (kD). The lability of h-TGFe to sodium dodecyl sulfate precludes any attempt to elute activity from polyacrylamide gels as has been done for the possible bovine counterpart of h-TGFe See, Halper and Moses, Cancer Research, 47, 4552-4559 (1987). However, the lability of h-TGFe to sodium dodecyl sulfate suggests that structural differences exist between hTGFe and the bovine molecule described by Halper and Moses (1987), supra.

Preliminary purification of h-TGFe from 25 liters of SW13 conditioned medium was carried out using reverse phase (C18) and cation exchange HPLC (carboxymethyl). Although two peaks of activity were recovered from the large scale C18 column it is likely that the second (more hydrophobic) peak is an artifact of the HPLC process, since upon treatment with 8M urea and fractionation on a BioGel P60 column, the second peak co-elutes with the Peak I activity. This result suggests that the Peak II material is an aggregate of h-TGFe with unidentified proteins. The behavior of h-TGFe on a carboxymethyl cation exchange column indicated an apparent pI close to that of trypsinogen (approximately 9.3). Specifically, the pI of h-TGFe as measured by cation exchange chromatography in the presence of 8M urea was approximately 8.5-10. These results show that cation exchange and reverse phase methods are useful in the purification of h-TGFe.

This invention also relates to a method of producing h-TGFe which comprises:

a) culturing, in the absence of serum, cells or a cell line which is capable of producing h-TGFe in conditioned medium in the absence of serum; and

b) isolating the h-TGFe produced from such culturing. Preferably, the method of producing h-TGFe includes the following steps:

a) culturing, in the absence of serum, cells or a cell line which is capable of producing h-TGFe in the absence of serum;

b) collecting the medium, preferably after the cells have grown to confluence;

c) sequentially subjecting the medium to reverse phase chromatography, size exclusion chromatography and cation exchange high pressure liquid chromatography; and

d) collecting the fractions containing h-TGFe.

This invention also relates to the h-TGFe produced according to the method of this invention.

As outlined above, the purification process of this invention comprises three steps which can be used for isolating and purifying h-TGFe from an impure solution thereof. These steps comprise:

a) collecting conditioned medium of cells capable of producing h-TGFe, preferably after the cells have grown to confluence;

b) sequentially subjecting the conditioned medium to reverse phase chromatography, size exclusion chromatography and cation exchange high pressure liquid chromatography; and

c) collecting the fractions containing h-TGFe. This invention also relates to the h-TGFe purified according to the method of this invention.

Each of the steps of the production or purification process of the invention can be combined with other process steps to arrive at a complete process for isolating and purifying h-TGFe from a producing recombinant or native, prokaryotic or eukaryotic, cell culture.

Conditioned medium is collected (harvested) by decanting from a batch culture system or by collecting medium from a flow-through system. The medium is optionally clarified such as by filtration and/or centrifugation, and the h-TGFe is initially isolated by standard techniques, e.g., selective precipitation, or, preferably, adsorption chromatography. This step results in isolation of most of the h-TGFe while substantially reducing the volume of medium for further processing. Adsorption chromatography employs a solid support capable of handling a large volume of medium at a high flow rate and with high efficiency. An example of such adsorption chromatography is chromatography using an affinity ligand such as polyclonal or monoclonal antibodies, cation exchangers such as S-sepharose, a metal chelating agent, wheat germ agglutinin, and various dyes. The preferred collection step is ion exchange chromatography. The support can be, e.g., a soft gel such as a dextran, agarose or polyacrylamide gel, a rigid gel such as Fractogel® vinyl polymer gel, 32-63 um size (Pierce Chemical Co., Rockford, Illinois) or any other support capable of adsorbing h-TGFe which is stable below pH 5.

In carrying out the collection step, washing is preferably carried out at pH 2-5, preferably at pH 4.0. The buffering agent can be any water-soluble acid/salt, for example, acetic acid, adipic acid, citric acid, lactic acid, tartaric acid, aspartic acid and glutamic acid, and their salts. The preferred buffer is ammonium acetate.

To elute the h-TGFe, a solution of an inorganic alkali or alkaline earth metal salt or of a chaotrope at <pH 5, e.g., pH 4.0, is used. The preferred elution solution is 0.5 to 2.5 M, preferably 2.0 M, sodium chloride in 0.05 M ammonium acetate (pH 4.0).

The impure solution obtained from the collection step is preferably further partially purified prior to reverse-phase chromatography. The partial purification can be accomplished by any means including, for example, by selective precipitation, affinity chromatography, ultrafiltration, normal phase chromatography and size exclusion, or any combination of these or other steps. Preferably, the impure solution has been partially purified such that the specific activity of h-TGFe is at least about 5 fold and preferably about 10 to 20 fold greater than the starting material, based on total protein content and biological activity in the SW13 soft agar assay prior to the reversed phase procedure.

Illustrative of process steps for achieving a desired degree of purity prior to the reverse phase procedure are ultrafiltration using, e.g., YM5 membranes (Amicon, Danvers, Massachusetts, U.S.A.) followed by size exclusion chromatography on, e.g., BioGel P60 (Bio Rad, Rockville Center, New York, U.S.A.). Such chromatography is carried out at a pH lower than 4 and preferably employs acetic acid (1M) and is carried out in the presence of a chaotropic agent such as 8M urea. Elution is accomplished under the same conditions.

In the preferred process of the invention, the partially purified solution from the collection step which has been treated by ultrafiltration and by a size exclusion chromatography step is then treated by reverse-phase chromatography.

Reverse-phase chromatography utilizes a hydrophobic support to adsorb a protein from an aqueous solvent, followed by selective elution with a polar, organic solvent, typically an alcohol-water mixture. The polar organic solvent causes elution by displacing hydrophobic domains on the polypeptide. Many proteins cannot be purified in this way due to the use of harsh conditions which can cause a protein, especially a protein for which the activity is conformation dependent, to lose activity.

The reverse-phase chromatography is carried out, typically, using an alkylsilica support, although any hydrophobic support can be employed, e.g., alkyl substituted carbohydrate or alumina gels. The preferred support is a $C_2$ to $C_{18}$ bonded silica, preferably $C_4$. Other silanes include octyldimethyl, octadecyldimethyl, phenyldimethyl, actyl, octadecyl and phenyl silanes. Particle size can be 5-100 um, preferably about 15 20 um. Typical pore size is 200 to 500 A, preferably 300 A.

The impure solution prior to the reverse-phase chromatography step preferably is adjusted to a very low pH, e.g., 1.5 to 3.5, with, e.g., heptafluorobutyric acid, acetic acid, phosphoric acid or trifluoroacetic acid. Inclusion of a chaotrope or an organic solvent in the impure solution, e.g., 8 M urea, improves separating, apparently by disassociating h-TGFe aggregates. Elution is typically accomplished by adding increasing amounts of an organic solvent to diminish hydrophobic interactions, e.g., acetonitrile, dioxane, tetrahydrofuran or a water-miscible alcohol, e.g., ethanol, methanol, n-propanol or isopropanol. The preferred elution gradient is 0-50% acetonitrile containing 0.05% trifluoroacetic acid.

Following the reverse-phase chromatography, three more steps are desirable to produce a more pure h-TGFe. These steps are ion exchange chromatography, preferably in the presence of a chaotrope, reverse phase high pressure liquid chromatography (HPLC) and affinity chromatography, preferably in red sepharose.

The ion exchange step serves to concentrate the h-TGFe and to remove the organic solvent. Preferably, this procedure is carried out using a cation exchange support, such as a carboxymethyl or sulfopropyl support equilibrated with a buffering solution such as 0.05 M ammonium sulfate (pH 4) and a chaotrope such as 8 M urea. Material is eluted from the support by increasing the concentration of salt, e.g., from 0.05 M to 1 M, while keeping the concentration of the chaotrope constant. The reverse phase step removes salts and urea prior to affinity chromatography. It can be carried out as described above, except that the preferred particle size is 5μm and the preferred stationary phase is phenylsilane. The red sepharose step is carried out by lyophilization of active proteins from the reverse phase step, followed by reconstitution in a buffering agent, e.g., 0.05M sodium phosphate (pH 7), containing a low concentration of a salt, e.g, 0.3 M sodium chloride. The column typically contains 1 ml of red sepharose (Pharmacia). After loading the reconstituted h-TGFe, the column is washed with a buffered salt preparation, e.g., 1 M sodium chloride in 0.05 M sodium phosphate (pH 7.0). It is then washed with an acidic solution, e.g., 0.05% trifluroracetic acid, before being eluted with a combination of high salt and organic solvent, e.g., 1 M ammonium acetate in 50% n-propanol. It is often desirable to further purify the eluted material using a reverse phase step as described above to remove salt and any red dye that may have detached from the column.

Each process step of the invention, as noted previously, can be combined with other process steps which may or may not be process steps of the invention.

This invention is also related to a recombinant DNA molecule, such as a cDNA, which contains the h-TGFe coding sequence. Such cDNA can be prepared by conventional techniques such as by reverse transcription of mRNA isolated by conventional techniques from a cell or culture producing h-TGFe, such as by the method of Han et al., Biochemistry, 26, 1617-1625 (1987). As stated above, the term 'h-TGFe coding sequence' encompasses the wild-type h-TGFe coding sequence as well as all functional mutants and derivatives of the native h-TGFe coding sequence. By the term 'functional mutants and derivatives of the native h-TGFe coding sequence' is meant any coding sequence, such as a fusion of the h-TGFe coding sequence to another protein's coding sequence, as well as a native h-TGFe coding sequence encompassing substitutions, deletions or additions of amino acids, which encodes a protein which has substantially the same biological activity as native h-TGFe, i.e., a protein which has the ability to induce the growth of epithelial cells. Such functional mutants and derivatives are conventionally prepared by one of skill in the art employing, e.g., site directed mutagenesis, random mutagenesis, chemical synthesis of nucleotide or peptide sequences, truncation or deletion of part of the coding sequence for native h-TGFe, chemical modification of the above or concatenation of more than one segment of the h-TGFe coding sequence. Such functional mutants and derivatives of the h-TGFe coding sequence also include chemically modified forms thereof, e.g., glycosylated and alkylated forms which have substantially the same biological activity as native h-TGFe.

As a further embodiment of the recombinant DNA molecule of the invention, there is provided a recombinant DNA vector comprising the coding sequence of this invention. This vector preferably contains the above coding sequence in operative association with suitable expression control sequences. By 'suitable expression control sequences' is meant those sequences which regulate the transcription and translation of a structural gene. Such expression control sequences are well

Still a further aspect of this invention is a host cell transformed with the vector of this invention. Such host cell is capable of growth in a culture medium and is capable of expressing the coding sequence of the invention. Such host cell is prepared by the method of this invention, i.e., transforming a desired host cell with the vector of the invention. Such transformation is accomplished by utilizing conventional transformation techniques. Host cells which may be suitable include, but are not limited to, mammalian cells, insect cells, bacterial cells, plant cells and fungal cells. Thus, this invention is not limited to any specific host cells.

The present invention also relates to a method of producing the protein encoded by the coding sequence of this invention which comprises culturing the transformed host of the invention in appropriate culture media and isolating such protein. By "appropriate culture media" is meant that media which will enable the transformed host to survive and which will also enable such host to express the coding sequence of the invention in recoverable quantity. It will be appreciated by one of skill in the art that the appropriate culture media to use will depend upon the host cell employed. The isolation of the protein so produced is accomplished from a culture lysate of the host or, if appropriate, directly from the host's culture medium, and is carried out by conventional protein isolation techniques such as those discussed above.

This invention also relates to a synthetic DNA molecule containing the h-TGFe coding sequence. By the term 'synthetic' is meant a DNA molecule prepared by synthetic rather than recombinant techniques. Such techniques are well known in the art using commercially available DNA synthesizers. As stated above, the term 'h-TGFe coding sequence' encompasses the wild-type h-TGFe coding sequence as well as all functional mutants and derivatives of the native h-TGFe coding sequence. By the term 'functional mutants and derivatives of the native h-TGFe coding sequence' is meant any coding sequence which encodes a protein which has substantially the same biological activity as native h-TGFe, i.e., a protein which has the ability to induce the growth of epithelial cells. Such functional mutants and derivatives of the h-TGFe coding sequence also include chemically modified forms thereof, e.g., glycosylated and alkylated forms which have substantially the same biological activity as native h-TGFe.

The present invention also relates to a method of producing the protein encoded by the coding sequence of this invention which comprises introducing the coding sequence of this invention into mammalian embryos, preferably in association with signal sequences that direct the secretion of h-TGFe into milk, growing the embryos to maturity and inducing them to produce milk, and isolating the protein so produced. The above method can be accomplished by one of skill in the art using published methods such as the method of Gordan et al., Biotechnology, 5, 1183-1187 (1987). The protein produced can be purified from the milk using techniques similiar to those described above.

The present invention also relates to the protein encoded by the coding sequence of this invention. Such protein may be prepared by the method of this invention. Alternatively, such protein may be conveniently prepared by one of skill in the art by any conventional protein preparation techniques such as oligonucleotide synthesis.

This invention also relates to any polyclonal or monoclonal antibody which is reactive against h-TGFe . Such antibodies are prepared utilizing conventional techniques, e.g., polyclonal antibodies may be prepared according to the method described in 'Methods in Immunology", 3rd edition, ed. J. Garrey et al., published by W. A. Benjamin Inc., pages 189-213 (1987), and monoclonal antibodies may be prepared according to the method of Kohler and Milstein, Nature, 256, 495 (1975).

This invention is also related to any polyclonal or monoclonal antibody which is reactive against any h-TGFe polyclonal or monoclonal antibody, i.e., any anti-idiotypic antibody. Anti-idiotype antibodies are conventionally prepared by one of skill in the art, e.g, by the method of Kunkel et el., Science, 140, 1218-1219 (1983) or Sege and Peterson, Proc. Natl. Acad. Sci., U.S.A., 75, 2443-2447 (1978).

This invention also relates to a method of stimulating the growth of epitheial cells in a human in need thereof which comprises administering an epithelial cell stimulating amount of h-TGFe to such human. h-TGFe is a substance capable of inducing growth of human epithelial cells. Therefore, it is especially useful in clinical situations where the proliferation of epithelial cells is required, e.g., wound healing, treatment of ulcers and burns, and re-epithelialization of tissues following radiotherapy. Since h-TGFe does not stimulate the growth of human fibroblastic cells, e.g., 3T3 cells, administration of h-TGFe according to the method of this invention is not expected to produce an undesirable fibrotic response. Several other growth factors which may also induce the growth of human epithelial cells, e.g., EGF, FGF and Platelet-derived growth factor (PDGF), do stimulate fibroblastic cells, and thus, their administration may produce an undesirable fibrotic response. Furthermore, h-TGFe is of human origin and, therefore, is unlikely to produce an undesirable immune response upon administration according to the method of this invention. Modes of administration of h-TGFe include topical application; parenteral administration, e.g.,

injection intravenously, subcutaneously or intramuscularly via application directly into injured tissue; and via mucous membranes, e.g., aerosol inhalation, nasally, vaginally, rectally, enterally (via the mucosal membrane of the intestines), or buccally. For topical application, a dose range of from about 0.01 to about 10 mg/M² of body surface per application is appropriate. One of skill in the art will appreciate that the number of topical applications per day or per course of treatment will depend on a number of factors, such as, but not limited to, the severity of the condition being treated, the surface area of the condition to be treated and the age of the individual being treated. For parenteral administration, a dose range of from about 1 to about 100 mg/M² of body surface/day is appropriate. One of skill in the art will appreciate that the number of parenteral applications per day or per course of treatment will depend on a number of factors, such as, but not limited to, the severity of the condition being treated and the age and weight of the individual being treated. For administration via mucous membranes, a dose range of from about 0.01 to about 10 mg per application is appropriate. One of skill in the art will appreciate that the number of applications administered via mucous membranes per day or per course of treatment will depend on a number of factors, such as, but not limited to, the severity of the condition being treated and the age and weight of the individual being treated.

This invention also relates to a pharmaceutical composition comprising an effective amount of human epithelial cell transforming growth factor (h-TGFe) and a pharmaceutically acceptable carrier or diluent. Such compositions may be prepared for use for parenteral (subcutaneous, intramuscular or intravenous) administration, particularly in the form of liquid solutions or suspensions; for use by vaginal or rectal administration particularly in semi-solid forms such as creams and suppositories; or intranasally, particularly in the form of powders or nasal drops.

As an example of a pharmaceutical composition of the invention suitable for intranasal administration, preparation may be effected by filling any conventional intranasal administration container with the parenteral pharmaceutical solution described above. The compositions may conveniently be administered in unit dosage form and may be prepared by any of the methods well known in the pharmaceutical art, for example as described in Remington's Pharmaceutical Sciences, Mack Publishing Company, Easton, PA., 1970. Formulations for parenteral administration may contain as common excipients sterile water or saline, polyalkylene glycols such as polyethylene glycol, oils of vegetable origin, hydrogenated naphthalenes and the like. Formulations for vaginal or rectal administration, e.g. suppositories, may contain as excipients, for example, polyalkyleneglycols, petroleum jelly, cocoa butter, and the like. Formulations for administration in the form of nasal drops. Formulations include incorporation into creams, lotions or suspensions of slow release microcapsules, solutions in sterile saline (for example for instillation into the eye following corneal injury), impregnation of absorbent pads and bandages or covalent crosslinking to collagen fibers or pads used in surgery. Formulations may include other growth factors (such as epidermal growth factor, transforming growth factor type alpha) tumor necrosis factor, fibroblast growth factors and insulin, as well as protease inhibitors, vasoconstrictors, etc. to prolong the duration of action at the injury site. As an example, a topical pharmaceutical composition of this invention may be prepared by dissolving the desired amount of h-TGFe in water, and adding the necessary amounts of methyl and propyl parabens, Tween 80 and sorbitol to form a solution, and such solution is heated at approximately 50°C and added to a separate solution containing the necessary amount of cetyl alcohol, stearyl alcohol and Span 20 dissolved in mineral oil and also heated at approximately 50°C, and the two solutions are mixed until emulsification takes place, and the preparation is stirred until it cools, and then it is filled into ointment tubes or jars. As an example of a parenteral pharmaceutical composition of the invention, the desired amount of h-TGFe is dissolved in sterile water for injection, the required amounts of buffering agents, e.g., sodium phosphate to 0.05M, and sodium chloride are added to adjust the pH to 7.4 and render the solution isotonic, and the solution is aseptically filtered and then filled into ampules suitable for parenteral administration. As an example of a pharmaceutical composition of the invention suitable for intranasal administration, any conventional intranasal delivery container may be filled with the parenteral pharmaceutical compostion described above.

Without further elaboration, it is believed that one of skill in the art can, using the preceding description, utilize the present invention to its fullest extent. The following Examples are, therefore, to be construed as merely illustrative and not a limitation of the scope of the present invention in any way.

### EXAMPLES

A. Materials and methods

## 1. Cell cultures and growth factors

All cell lines used were obtained from American Type Culture Collection, Rockville, Maryland, U.S.A. and were shown to be free from mycoplasma contamination using the Gen Probe assay (Gen Probe, San Diego, CA 92123) and broth culture (Microbiological Associates, Bethesda, Maryland). Isoenzyme analysis demonstrated that the SW13 cells were of human origin (Corning Authentikit). Murine epidermal growth factor (EGF), human platelet-derived growth factor (PDGF), bovine pituitary fibroblast growth factor (FGF) and bovine insulin were supplied by Collaborative Research, Lexington, Massachusetts 02173). Synthetic human gastrin and bombesin were obtained from Sigma, St. Louis, Missouri. Human platelet TGF-$\beta$ was a gift from Dr. R. Assosian, Columbia University, New York. The biological activities of EGF and TGF-$\beta$ were verified using growth of NRK-49F cells in soft agar as described by Todaro et al., Proc. Natl. Acad. Sci., 77, 5258-5262 (1980). PDGF was active in the NIH-3T3 thymidine uptake assay described by Bowen-Pope et al., J. Biol. Chem., 257, 5161-5171 (1982).

## 2. Collection of conditioned media

Cells were grown to confluence in Dulbecco's modified Eagles Medium (DMEM) (Gibco, Grand Island, New York, 14072) supplemented with 10% fetal bovine serum, in roller bottles. The cells were washed with phosphate buffered saline (PBS), incubated in serum-free DMEM for 4-5 hours (hr) and again washed with PBS. Each roller bottle received 200 mL of serum-free, phenol red-free RPMI 1640 medium (Gibco, Grand Island, New York, 14072) supplemented with 10 $\mu$g/L (final concentration) freshly prepared ferrous sulfate and 5 ng/ml (final concentration) sodium selenite (Collaborative Research, Lexington, Massachusetts). Cells were incubated with the serum-free medium for 2 days and the medium was collected, acidified with 5 ml/liter trifluoroacetic acid (TFA) and stored at 4° C. The acidified conditioned medium was centrifuged at 500 X g for 15 minutes (min), filtered through a 0.45 $\mu$m membrane (Gelman, Ann Arbor, Michigan, 48106), and biological activity was determined after concentration by ultrafiltration (Amicon YM5 membrane, 5000 Mr cutoff, Amicon, Danvers, Massachusetts, 01923) and dialysis against 0.05% TFA. Mr means relative molecular mass. The following cell lines were tested for production of activity: SW13, SW48, SKC01, LOVO, CACO 2, HT-29, COLO 320DM, COLO 320HSR, SW948, DLD-1 NRK-52E, HCT-15, WIDR, SW403 and SW742.

## 3. Biological assays

h-TGFe activity was measured using growth of SW13 cells in soft agar as described by Halper et al., Cancer Research, 43, 1972-1979 (1983), except that the base and upper layers contained 0.5% and 0.3% agarose, respectively in DMEM, 10% fetal bovine serum, and 5000 cells were plated per 3 cm dish.

Growth of fetal bovine heart endothelial (FBHE) cells in monolayer culture by the method of Gospodarowicz et al., J. Biol. Chem., 250, 2515 (1975), was used to measure FGF activity. Samples were added to 3 cm dishes containing 2 mL DMEM, 10% fetal bovine serum. Each dish then received 100 $\mu$l of a suspension of 5 x 10$^4$ FBHE cells in DMEM, 10% fetal bovine serum. Cultures were incubated for 5 days, fixed with 10% buffered formalin and stained with 0.5% crystal violet. Cell growth was quantitated by measurement of the total colony areas for each dish by image analysis.

## 4. Analysis of h-TGFe activity in human milk and tissue extracts

Human milk was delipidated by ether extraction and acidified with TFA (0.5% final concentration). The acidified material was filtered through a 0.45 $\mu$m membrane and concentrated 5-fold using an Amicon YM5 membrane. The concentrate was dialyzed against 0.05% TFA, lyophilized, reconstituted at 10 times the original concentration and bioassayed on SW13 cells.

Samples of colon carcinoma tissue from patients along with two specimens of normal adjacent colon were obtained at surgery and snap frozen in liquid nitrogen. One gram of tissue was thawed and homogenized in 4 mL acidified ethanol containing pepstatin and phenylmethyl sulfonyl fluoride as described by Roberts et al., Proc. Natl. Acad. Sci., 82, 119-123 (1985). The homogenates were centrifuged at 12000 X g for 25 min and supernatants were dialyzed against 0.05% TFA and lyophilized. Extracts were reconstituted in 1 mL 0.05% TFA for bioassay.

## 5. Methods utilized for characterization of SW13-derived TGFe

### a. Trypsin sensitivity

Trypsin sensitivity of h-TGFe was assessed by incubation of a 10X concentrate of SW13 conditioned medium with 50 $\mu$g/mL trypsin (Sigma) for 2 hr at 37° C, pH 8.5. As controls, 500 $\mu$g/mL (final

concentration) ovine trypsin inhibitor (Sigma) was added per mL of sample before enzyme treatment and a further sample was treated with inhibitor only. After 2 hrs the reaction was stopped by addition of trypsin inhibitor and the samples were bioassayed.

b. Heparin Affinity

The affinity of h-TGFe for heparin was determined by passage of crude conditioned medium reconstituted in 50 mM $NaH_2PO_4$, 0.25 M NaCl pH 7.0 through a 0.5 x 3 cm column packed with 1 gm of heparin-sepharose conjugate (Pharmacia, Piscataway, New Jersey 08854). Samples of the starting material and the column effluent were bioassayed on SW13 cells. As a control, 50 ng/mL FGF in the same buffer was passed through the same column and samples were bioassayed using FBHE cells. h-TGFe was itself bioassayed against FBHE cells and on NIH-3T3 cells using the thymidine uptake assay described by Bowen-Pope et al., J. Biol. Chem., 257, 5161 5171, (1982), with PDGF as positive control.

c. Denaturing agent sensitivity

The sensitivity of h-TGFe to denaturing agents was tested by incubating crude conditioned medium concentrate with one of 0.1% Tween 80 (Sigma), 2% sodium dodecyl sulfate, 0.005 M dithiothreitol or 8M urea for 1 hr. The treated samples, along with an untreated control sample were exhaustively dialyzed against 0.05% TFA to remove the denaturing agent and bioassayed on SW13 cells.

d. Molecular Weight

The molecular mass of h-TGFe was estimated by gel filtration chromatography of conditioned medium concentrate on a BioGel P60 column in the presence of 8M urea. Lyophilized concentrate was dissolved in 20 mL 8M urea in 0.05% TFA and applied to 5 x 86 cm column (Pharmacia, Piscataway, New Jersey) containing BioGel P60 resin (BioRad, Rockville Center, New York 11571). The flow rate was 36 mL/hr, 20 mL fractions were collected and 100 $\mu$L aliquots were dialyzed against 0.05% TFA to remove urea and bioassayed. Molecular mass standards were ovalbumin (43k), chymotrypsinogen (25k) lysozyme (14.3k), insulin (6k) and vitamin B12 (1.3k).

e. Determination of the isoelectric point by cation exchange chromatography in the presence of 8M urea

Conditioned medium from SW13 cell culture was concentrated by capture on S-sepharose (Pharmacia, Piscataway, New Jersey), elution with 2M sodium chloride, dialysis and lyophilization. This material was reconstituted in 0.05 M ammonium acetate pH 4.0 containing 8M urea as a dissociating agent and loaded onto a 5 x 0.5 cm column of Mono S (Pharmacia, Piscataway, New Jersey) equilibrated in ammonium acetate/8M urea as above. Proteins were eluted from the column using a linear gradient of 0.05-0.5 M ammonium acetate pH 4.0 in 8M urea, increasing at 0.015 M/minute. The flow rate was 1 ml/minute and 1 ml fractions were collected. Aliquots (5$\mu$1) of each fraction were assayed for activity on SW13 cells in the soft agar assay. The column was calibrated by running standard proteins of known pl under the same conditions, with detection by absorbance at 280 nm. The standards, were $\beta$ lactoglobulin (pl 5.2) bovine carbonic anhydrase B (5.85) human carbonic anhydrase (6.55), horse myoglobin (6.85, 7.35) lentil lectin (8.15, 8.45, 8.65), trypsinogen (9.3) and cytochrome C (10.25).

6. Purification of h-TGFe

h-TFGe was purified from SW13 conditioned medium by sequential reverse phase, size exclusion and cation exchange HPLC. A 25 liter batch of acidified conditioned medium was loaded at 75 mL/min onto a 5 x 30 cm preparative column containing Vydac 218TP C18 packing (HP Chemicals, StN Louis, Missouri 63108), particle size 15-20 $\mu$m, using a Waters Delta-Prep system (Millipore, Milford, Massachusetts 01757). The column was washed with 0.05% TFA until the absorbance at 214 nm reached baseline and a gradient of 0.05% TFA in acetonitrile was run at 0-30% over 50 min, 30-50% over 20 min and 50-100% over 10 min at a flow rate of 70 ml/min. Fractions were collected every 2.5 min, acetonitrile was removed by vacuum evaporation and aliquots were bioassayed on SW13 cells.

Active fractions from the reverse phase step were concentrated tenfold using Amicon YM5 membranes and concentrates were loaded onto two series connected TSK 3000 SW size exclusion columns (each 2.5 x 60 cm) (Beckman, Fullerton, California). Material was eluted from the columns with 0.05% TFA at a flow rate of 2 ml/min and 4 mL fractions were collected.

Sodium phosphate was added to the active material from the size exclusion step to a final concentration of 20 mM. The pH was adjusted to 6.0 with sodium hydroxide and the samples were centrifuged at 90,000 x g for 30 min. Supernatants were loaded onto a CM2SW cation exchange column (Beckman, Fullerton, California) and washed with 20 mM phosphate buffer pH 6.0. A linear gradient of 0-0.6M sodium chloride in phosphate buffer pH 6.0 was run at 0-95% over 90 min and 2 mL fractions were collected and bioassayed.

An active sample of h-TGFe from the cation exchange step was analyzed by reverse phase HPLC. The sample was acidified to pH 2.5 with 1% TFA and loaded onto a 0.46 x 30 cm column containing Vydac C18 218TP 10 μm packing (The Nest Group, Southboro, Massachusetts 01772). A gradient of 0-50% 0.05% TFA in acetonitrile was run at 0.25%/min and 2 mL fractions were collected and bioassayed after removal of the acetonitrile by vacuum evaporation. Recovery of activity during purification was monitored by constructing dose-response curves using the purified material. One unit of activity was defined as that required for a 50% maximal response. Protein concentration was measured by the BCA method (Pierce, Rockford, Illinois 61105), and by electronic integration of absorbance peaks using ovalbumin as a standard.

## RESULTS

### 1. Production of h-TGFe activity by human tissues, human milk and cell lines

Extracts of two human colon carcinomas examined in the soft agar assay (Halper et al., Cancer Research, 43, 1972-1979 (1983)) contained h-TGFe activity, whereas normal adjacent colon tissue from these patients had less activity. Activity was also detected in human milk. Of the cell lines tested, SW13, SW403, LOVO, NRK-52E, WIDR and HCT-15 released h-TGFe activity into the culture medium. Smaller but detectable amounts were produced by SKCO1 and SW48 cells, while DLD-1, HT-29, SW948, COLO 320 (DM or HSR), CACO 2 and SW742 failed to release observable amounts of h-TGFe.

### 2. Relationship between h-TGFe and other growth factors and affinity of h-TGFe to bind to heparin

When SW13 conditioned medium was concentrated 50-fold using ultrafiltration and dialyzed against 0.05% TFA, the resulting material produced a dose-related increase in colony formation by SW13 cells in the soft agar assay (Halper et al., Cancer Research, 43, 1972-1979 (1983)). The ED50 of this crude concentrate was approximately 37 μl (corresponding to 73 μg of protein) per dish, giving a specific activity of 13.7 half-maximal units per mg protein. This activity was sensitive to 50 μg/mL trypsin and was not mimicked by EGF, PDGF, gastrin, bombesin, insulin or TGF-β. However, FGF produced a dose-related increase in colony formation.

To further examine the possible relationship between h-TGFe and FGF, a sample of conditioned medium concentrate was passed through a heparin-sepharose column. Most of the h-TGFe was recovered in the column breakthrough fraction, indicating that it had little, if any, affinity for heparin. In contrast, FGF applied to the same column was completely retained, as expected from the known affinity of FGF-like growth factors for heparin as reported by Lobb et al., J. Biol. Chem., 261, 1924-1928, (1986). The biological activity of h-TGFe could also be separated from that of FGF. When an aliquot of h-TGFe from the reverse phase purification step (see below) was assayed on FBHE cells, no stimulation of proliferation was observed, in contrast to an 8 to 9-fold increase in cell growth produced by FGF. When FGF was assayed on FBHE cells in the presence of h-TGFe this proliferative response was not diminished, indicating that the absence of effect of h-TGFe alone was not due to contamination of the samples with inhibitors or toxins. Furthermore, partially purified h-TGFe did not stimulate thymidine uptake in quiescent 3T3 cells in contrast to PDGF.

### 3. Effects of denaturing agents on h-TGFe activity

Crude conditioned medium concentrate from SW13 cells was incubated with 8M urea, 0.005 M dithiothreitol, 2% sodium dodecyl sulfate or 0.01% Tween 80 at room temperature for 1 hr. After removal of the denaturing agent by dialysis, bioassay in the soft agar assay (Halper et al., Cancer Research, 43, 1972-1979 (1983)) indicated that the h-TGFe is stable to 8M urea or Tween 80 but labile to 0.065M dithiothreitol or sodium dodecyl sulfate. In addition, the h-TGFe was found to be labile to 0.1 g/100 mL tetrabutylammonium phosphate after bioassay in the soft agar assay (Halper et al., Cancer Research, 43, 1972-1979 (1983)).

### 4. Effect of Trypsin on h-TGFe

THe sensitivity of h-TGFe to trypsin was assessed by the method indicated supra, and the results indicated that h-TGFe activity is inactivated by 50 μg/mL of trypsin.

## 5. Purification of h-TGFe

A 25 liter batch of SW13 conditioned medium was applied to a preparative column containing Vydac 218TP packing (HP Chemicals, St. Louis, Missouri 63108), particle size 15-20 μm, and eluted with an acetonitrile gradient. Two peaks of activity were observed in the resulting fractions after bioassay in the soft agar assay (Halper et al., Cancer Research, 43, 1972-1979 (1983)): Peak I eluted at 20-30% acetonitrile and Peak II at 35-40%. Fractions 17-20 (Peak I) and 23-26 (Peak II) respectively were pooled, concentrated and applied to a size exclusion column. Activity eluted at apparent molecular mass of 59 kD (Peak I) and 35-45 kD (Peak II). When a sample of crude conditioned medium of Peak II material was pretreated with 8M urea to dissociate non-covalently bound proteins and fractionated on a size exclusion column, a single peak of activity was observed at an apparent molecular mass of 59 kD.

Further purification of Peak I material was obtained by HPLC on a CM2SW cation exchange column (Beckman, Fullerton, California). h-TGFe eluted at 0.3 M NaCl and was subsequently run on an analytical reverse phase column. Elution was effected using a linear gradient of 0-50% acetonitrile increasing at 0.25%/minute. h-TGFe eluted as a single peak at approximately 20% acetonitrile (flow rate-1 mL/minute). This peak was well separated from the majority of contaminating protein as judged by the UV absorbance profile.

The purification statistics show excellent recovery of h-TGFe from SW13 conditioned medium by preparative reverse phase HPLC. Over 80% of the total activity was distributed in roughly equal amounts between Peaks I and II. Recovery was 50% of total after the size exclusion step but only 11% of the original activity was present after cation exchange HPLC. Nevertheless, this step resulted in greater than 100-fold purification of the material from the size exclusion column. Insufficient material was present after the analytical reverse phase step to determine recovery or degree of purification; however, a rough estimate of activity suggest a specific activity of 30000 U/mg. One 'U' is a unit, and one unit is the amount of h-TGFe (either in mg/protein or in μL of sample/mL of culture medium) required to give a 50% of maximal response in the soft agar assay of Halper et al. (1983), supra. This figure, i.e., 30000 U/mg, corresponds to a greater than 2000-fold purification at this stage.

## Claims

1. A human epithelial cell transforming growth factor (h-TGFe) which has the following characteristics:

(a) stimulates anchorage independent growth of SW13 cells in soft agar in an autocrine fashion;

(b) is stable to pH 2-4, 8M urea and 0.01% Tween 80, but is labile to 5 μg/mL trypsin, 0.065M dithiothreitol, 0.1 g/100 mL tetrabutylammonium phosphate and 2% sodium dodecyl sulfate;

(c) does not bind to heparin-sepharose conjugate but does bind to red sepharose;

(d) fails to stimulate the growth of fetal bovine endothelial cells in monolayer culture;

(d) has a molecular mass of approximately 59 kilodaltons as measured by size exclusion gel permeation chromatography in the presence of 8M urea;

(f) has an isoelectric point of approximately 8.5-10 as measured by cation exchange chromatography in the presence of 8M urea;

(g) elutes at 20-30% acetonitrile from a C18 reverse phase column; and

(h) is produced by SW13, SW403, LOVO, NRK-52E, WIDR, HCT-15, SKC01 and SW48 cell lines and is detectable in human milk.

2. A pharmaceutical composition comprising a human epithelial cell transforming growth factor (h-TGF3) according to claim 1 and a pharmaceutically acceptable carrier or diluent.

3. The pharmaceutical composition of claim 2 which is in a form suitable for administration via mucous membranes.

4. The pharmaceutical composition of claim 2 which is in a form suitable for parenteral administration.

5. The pharmaceutical composition of claim 2 which is in a form suitable for topical administration.

6. A human epithelial cell transforming growth factor (h-TGFe) according to claim 1 for use as a therapeutic agent.

7. A human epithelial cell transforming growth factor (h-TGFe) according to claim 1 for use in stimulating the growth of epithelial cells.

8. A method of producing h-TGFe which comprises:

(a) culturing, in the absence of serum, cells or a cell line which is capable of producing h-TGFe in conditioned medium in the absence of serum; and

(b) isolating the h-TGFe produced from such culturing.

9. The method of claim 8 which comprises:

(a) culturing, in the absence of serum, cells or a cell line which is capable of producing h-TGFe under serum free conditions;

(b) collecting the conditioned medium produced in step a, preferably after the cells have grown to confluence.;

(c) sequentially subjecting the conditioned medium to reverse phase chromatography, size exclusion chromatography and cation exchange high pressure liquid chromatography; and

(d) collecting the fractions containing h-TGFe.

10. A method of purifying h-TGFe which comprises:

(a) collecting conditioned medium of cells capable of producing h-TGFe, preferably after the cells have grown to confluence;

(b) sequentially subjecting the conditioned medium to reverse phase chromatography, size exclusion chromatography and cation exchange high pressure liquid chromatography; and

(c) collecting the fractions containing h-TGFe.

11. A recombinant DNA sequence comprising the h-TGFe coding sequence.

12. The recombinant DNA sequence of claim 11 which is a cDNA containing the h-TGFe coding sequence.

13. The recombinant DNA vector containing the h-TGFe coding sequence operatively associated with an expression control sequence.

14. A host cell transformed with the vector of claim 13.

15. A method of producing a transformed host which comprises transforming a desired host cell with the vector of claim 13.

16. A method of producing h-TGFe which comprises culturing the transformed host of claim 14 in appropriate culture medium.

17. A polyclonal or monoclonal antibody which is reactive against h-TGFe.

18. A polyclonal or monoclonal antibody which is reactive against the h-TGFe antibody of claim 17.

19. A method of producing the protein encoded by the coding sequence of claim 11 which comprises introducing the coding sequence into mammalian embryos, growing the embryos to maturity and inducing them to produce milk, and isolating the protein so produced.

20. A synthetic DNA molecule containing the h-TGFe coding sequence.

Claims for the following Contracting States:GR, ES

1. A method of producing h-TGFe which comprises:

(a) culturing, in the absence of serum, cells or a cell line which is capable of producing h-TGFe in conditioned medium in the absence of serum; and

(b) isolating the h-TGFe produced from such culturing.

2. The method of claim 1 which comprises

(a) culturing, in the absence of serum, cells or a cell line which is capable of producing h-TGFe under serum free conditions;

(b) collecting the conditioned medium produced in step a, preferably after the cells have grown to confluence;

(c) sequentially subjecting the conditioned medium to reverse phase chromatography, size exclusion chromatography and cation exchange high pressure liquid chromatography; and

(d) collecting the fractions containing h-TGFe.

3. The method of claim 1 where the h-TGFe producing cell or cell line is selected from SW13, SW403, LOVO, NRK-52E, WIDR, HCT-15, SKC01 and SW48

4. A method of purifying h-TGFe which comprises:

(a) collecting conditioned medium of cells capable of producing h-TGFe, preferably after the cells have grown to confluence;

(b) sequentially subjecting the conditioned medium to reverse phase chromatography, size exclusion chromatography and cation exchange high pressure liquid chromatography; and

(c) collecting the fractions containing h-TGFe.

5. A method according to any one of claims 1 to 4 in which the h-TGFe produced has the following characteristics:

(a) stimulates anchorage independent growth of SW13 cells in soft agar in an autocrine fashion;

(b) is stable to pH 2-4, 8M urea and 0.01% Tween 80, but is labile to 5 $\mu$g/mL trypsin, 0.065M dithiothreitol, 0.1 g/100 mL tetrabutylammonium phosphate and 2% sodium dodecyl sulfate;

(c) does not bind to heparin-sepharose conjugate but does bind to red sepharose;

(d) fails to stimulate the growth of fetal bovine endothelial cells in monolayer culture;

(e) has a molecular mass of approximately 59 kilodaltons as measured by size exclusion gel permeation chromatography in the presence of 8M urea;

(f) has an isoelectric point of approximately 8.5-10 as measured by cation exchange chromatography in the presence of 8M urea;

(g) elutes at 20-30% acetonitrile from a C18 reverse phase column; and

(h) is produced by SW13, SW403, LOVO, NRK-52E, WIDR, HCT-15, SKC01 and SW48 cell lines and is detectable in human milk.

6. A method of producing a transformed host which comprises transforming a desired host cell with a recombinant DNA vector containing the h-TGFe coding sequence operatively associated with an expression control sequence.

7. A method of producing h-TGFe which comprises culturing the transformed host produced in claim 6 in appropriate culture medium.

8. A method of producing the protein encoded by the coding sequence of h-TGFe which comprises introducing the coding sequence into mammalian embryos, growing the embryos to maturity and inducing them to produce milk, and isolating the protein so produced.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CANCER SURVEYS, vol. 7, no. 4, 1988, pages 653-674; R. GREIG et al.: "Growth factors as a novel therapeutic targets in neoplastic disease" <br> * Page 663, last 5 lines of paragraph III,4 * <br> --- | 1-20 | C 07 K 15/00 <br> C 07 K 3/20 <br> A 61 K 37/02 <br> C 12 P 21/00 <br> C 12 P 21/08 <br> C 12 N 15/16 <br> C 12 N 1/00 |
| D,X | CANCER RESEARCH, vol. 47, 1st September 1987, pages 4552-4559; J. HALPER et al.: "Purification and characterization of a novel transforming growth factor" <br> * Whole document * <br> --- | 1-20 | |
| D,X | CANCER RESEARCH, vol. 43, May 1983, pages 1972-1979; J. HALPER et al.: "Epithelial tissue-derived growth factor-like polypeptides" <br> * Whole document" * <br> ----- | 1-20 | |

TECHNICAL FIELDS SEARCHED (Int. Cl.5)

A 61 K
C 12 N
C 12 P

The present search report has been drawn up for all claims

| Place of search | Date of completion of the search | Examiner |
|---|---|---|
| THE HAGUE | 15-02-1990 | ROSDY M.K.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)